(19) **Europäisches Patentamt / European Patent Office / Office européen des brevets**

(11) **EP 2 263 666 A1**

(12) **EUROPEAN PATENT APPLICATION**
published in accordance with Art. 153(4) EPC

(43) Date of publication:
**22.12.2010 Bulletin 2010/51**

(51) Int Cl.:
*A61K 31/198* (2006.01)   *A23L 1/305* (2006.01)
*A61P 1/04* (2006.01)   *A61P 1/06* (2006.01)

(21) Application number: **09720186.7**

(22) Date of filing: **11.03.2009**

(86) International application number:
**PCT/JP2009/054697**

(87) International publication number:
**WO 2009/113594 (17.09.2009 Gazette 2009/38)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **11.03.2008 JP 2008061769**

(71) Applicant: **Ajinomoto Co., Inc.**
**Tokyo 104-8315 (JP)**

(72) Inventors:
• **FUJITA, Shinichi**
**Kawasaki-shi**
**Kanagawa 210-8681 (JP)**

• **ISHIBASHI, Ikumi**
**Kawasaki-shi**
**Kanagawa 210-8681 (JP)**
• **OGAWA, Saori**
**Kawasaki-shi**
**Kanagawa 210-8681 (JP)**
• **SUZUKI, Manabu**
**Kawasaki-shi**
**Kanagawa 210-8681 (JP)**

(74) Representative: **Nicholls, Kathryn Margaret et al**
**Mewburn Ellis LLP**
**33 Gutter Lane**
**London**
**EC2V 8AS (GB)**

(54) **AGENT AND FOOD FOR PREVENTION/AMELIORATION OF FUNCTIONAL GASTROINTESTINAL DISORDERS**

(57)    Provided is an agent for the prophylaxis or improvement of functional gastrointestinal disorders, which contains glutamic acid or a salt thereof, and arginine or a salt thereof (excluding arginine glutamate) as active ingredients. The prophylactic or improvement agent can be produced conveniently and at low cost and is high safe and particularly effective for functional dyspepsia (FD) such as abdominal pain, heavy stomach, heartburn and the like, and upper gastrointestinal dysfunction such as esophageal reflux (GERD) and the like.

EP 2 263 666 A1

**Description**

Technical Field

The present invention relates to an agent for the prophylaxis or improvement of a functional gastrointestinal disorder. More particularly, the present invention relates to, an agent for the prophylaxis or improvement of functional gastrointestinal disorders (FGIDs), particularly, upper gastrointestinal dysfunctions such as functional dyspepsia (FD) (e.g., abdominal pain, heavy stomach, heartburn and the like), gastroesophageal reflux disease (GERD) and the like.

Background Art

Even with the advancement in endoscopic diagnosis, there are many cases where a complaint of the upper gastrointestinal symptoms such as upper abdominal pain, discomfort, postprandial heavy stomach, nausea, vomiting and the like cannot be fully explained. Such condition where a complaint of gastrointestinal symptom is reported but no organic disease is found by a general checkup including endoscopic examination, and no finding to elucidate the symptom is available is referred to as a functional gastrointestinal disorder, particularly functional dyspepsia (FD) or non-ulcer dyspepsia (NUD).
As diagnostic criteria for functional gastrointestinal disorder, Rome criteria are known, which were revised in April 2006 and published as Rome III in a journal of American Gastroenterological Association Journal (Gastroenterology). While the concept of FD does not change much, the dyspepsia symptoms in diagnostic criteria were simplified into four based on the results of the clinical studies since Rome II. They are bothersome postprandial fullness, early satiation, epigastric pain and epigastric burning, and FD is diagnosed when one or more symptoms therefrom are chronically (strictly not less than 3 months) seen, and organic diseases are excluded. On the other hand, in Japan, such case has been determined to be "upper abdomen gastrointestinal complaint associated with chronic gastritis" irrespective of organic findings, and, in clinical situations, diagnosed conventionally as "gastritis" or "chronic gastritis".
Even in cases where an organic disease (erosive esophagitis, peptic ulcer, acute gastritis, gastrointestinal cancer, pancreas/biliary disease etc.) is clearly observed, abdominal pain, discomfort, postprandial heavy stomach, nausea· vomiting and the like are also found. Accordingly, there is an urgent need for the improvement of such discomfortable feeling to ensure better QOL of patients. When NUD is joined with lower abdomen indefinite complaint such as defecation difficulty due to constipation, unrelieved feeling after defecation, abdominal pain, feeling of fullness in the abdomen and the like, about 30%-50% of the total population of Japan is assumed to have experienced some gastrointestinal indefinite complaint. The development of abdominal indefinite complaint is considered to be influenced by sex, aging, stress or overweight due to the western style diet, and is a disease representing the modern society along with the lifestyle-related diseases. Even though it is such a serious disease, the etiology of the gastrointestinal indefinite complaint is merely suggested to involve various diseases (chronic gastritis, diabetes, overweight, constipation etc.), and the only suggested mechanism of its onset is degraded gastrointestinal motility function. Taken together with the fact that the gastrointestinal motility function is degraded only in 50% of the total actual FD patients, it is clear that the developmental mechanism of FD has not been elucidated completely.
In addition, many of the patients with a progressive degenerative disease of the brain such as Parkinson's disease, Huntington chorea, olivopontocerebellar atrophy and the like, cerebral apoplexy and the like also develop gastrointestinal motility dysfunction, and improvement of QOL by the improvement of gastrointestinal motility function is considered to be necessary. It is considered that many of these patients cannot report indefinite complaint by themselves due to logopathy, disturbance of consciousness and the like. Thus, a care that removes a disturbance of sensation such as indefinite complaint and the like simultaneously with a care of organic dysfunction leads to the improvement of QOL in a true sense.
5-HT4 receptor agonists and the like have heretofore been used for the treatment of FD. For example, cisapride and metoclopramide have a prokinetic action on the stomach and intestines, and have been used for the treatment of the symptoms and the like of chronic gastritis, feeling of fullness in the abdomen, erosive esophagitis, abdominal indefinite complaint and pseudoileus. However, metoclopramide shows a side effect of extrapyramidal symptoms caused by the action on dopamine D2 receptors in the central nervous system, and cisapride has also been clarified to show parkinsonian symptoms. While mosapride etc. have also been used, the effect is not always sufficient, and side effects such as diarrhea and the like appear. In addition, although H2 antagonists and proton pump inhibitors are prescribed in many cases, the safety of long-term administration has not been established. Reports have also documented that long-term administration of proton pump inhibitor increases the risk of bone fracture and the like.
On the other hand, glutamic acid has been reported to be a prophylactic or improvement agent for functional gastrointestinal disorders. Furthermore, arginine glutamate has also been reported to be a prophylactic or improving agent for gastrointestinal tract disturbances (patent document 1). While arginine is suggested to possibly increase the onset frequency of myocardial infarction, the above-mentioned prophylactic or improving agent with a different mixing ratio of glutamic acid and arginine is not known.
patent document 1: WO2006/030980 A1

Disclosure of the Invention

Problems to be Solved by the Invention

The present invention aims to provide an agent for the prophylaxis or improvement of a functional gastrointestinal disorder, particularly, upper gastrointestinal dysfunction such as functional dyspepsia (e.g., abdominal pain, heavy stomach, heartburn and the like), gastroesophageal reflux disease and the like, which can be produced more conveniently and has high safety.

Means of Solving the Problems

The present invention has been made in view of the above-mentioned problems. The present inventors have found that administration of a preparation containing glutamic acid and arginine accelerates gastric emptying, and therefore, is effective for a functional gastrointestinal disorder associated with delayed gastric emptying. Moreover, it has been clarified that a similar effect can be exhibited even when the arginine ratio is changed, and they have found that the amount of arginine, which is a bitter substance and requiring masking for ingestion, can be reduced.

Accordingly, the present invention provides the following.

[1] An agent for the prophylaxis or improvement of a functional gastrointestinal disorder, comprising glutamic acid or a salt thereof, and arginine or a salt thereof, excluding arginine glutamate, as active ingredients.

[2] The agent of [1], wherein the functional gastrointestinal disorder is an upper gastrointestinal dysfunction.

[3] The agent of [2], wherein the upper gastrointestinal dysfunction is a functional dyspepsia or gastroesophageal reflux disease.

[4] The agent of any one of [1] to [3], wherein a molar ratio of the glutamic acid or a salt thereof and arginine or a salt thereof is 30:1 - 1:30.

[5] The agent of [4], wherein the molar ratio is 10:1 - 1:10.

[6] The agent of [4], wherein the molar ratio is 10:1 - 2:1 or 1:2 - 1:10.

[7] The agent of [4], wherein the molar ratio is 10:1 - 2:1.

[8] A medicament comprising the agent of any one of [1] to [7].

[9] A food comprising the agent of any one of [1] to [7].

[10] The food of [9], which is a food with health claims, food for specified health uses, food with nutrient function claims or a dietary supplement.

[11] A method of preventing or improving a functional gastrointestinal disorder, comprising administering an effective amounts of glutamic acid or a salt thereof, and arginine or a salt thereof, excluding arginine glutamate, to a subject affected with a functional gastrointestinal disorder.

[12] A method of treating a functional gastrointestinal disorder, comprising administering effective amounts of glutamic acid or a salt thereof, and arginine or a salt thereof, excluding arginine glutamate, to a subject affected with a functional gastrointestinal disorder.

[13] Use of glutamic acid or a salt thereof, and arginine or a salt thereof, excluding arginine glutamate, for producing the agents of [1] to [7].

[14] The agent of any one of [1] to [7], which is a solid preparation.

Effect of the Invention

According to the present invention, a medicament and a food useful for the improvement of functional gastrointestinal disorders, particularly functional dyspepsia, and upper gastrointestinal dysfunction such as esophageal reflux and the like can be provided. In addition, the medicament and food improve uncomfortable feeling caused by the above-mentioned diseases, enhance QOL of patients, and can be taken for a long time since only a few side effects are caused. When the amount of arginine, which is a bitter taste substance requiring masking on ingestion, is small, masking can be performed easily. On the other hand, when the amount of arginine is high, an effect of difficult induction of postprandial fullness and the like can be obtained in patients with a functional gastrointestinal disorder, particularly functional dyspepsia. In addition, a suppressive effect on symptoms caused by reflux can be achieved in patients with esophageal reflux.

According to the present invention, moreover, a medicament and a food can be produced with ease by merely blending glutamic acid (a salt thereof) and arginine (a salt thereof) without forming a salt and the like.

Brief Description of the Drawings

Fig. 1 shows the results of promotion of stomach emptying when the mixing ratio of glutamic acid and arginine is changed.
Fig. 2 shows the results of promotion of stomach emptying when the mixing ratio of glutamic acid and arginine is changed. Best Mode for Carrying out the Invention

The embodiment of the present invention is explained in the following.
The agent for the prophylaxis or improvement of functional gastrointestinal disorders of the present invention, which comprises glutamic acid or a salt thereof, and arginine or a salt thereof (excluding arginine glutamate) as active ingredients (hereinafter sometimes to be referred to as the prophylactic or improvement agent of the present invention) prevents the development of or improves the following symptoms in reproducible functional gastrointestinal disorders, particularly functional dyspepsia, and upper gastrointestinal dysfunction such as gastroesophageal reflux disease and the like, which decrease the QOL of patients.

The "functional gastrointestinal disorder" in the present invention refers to the state of decreased motility function of gastrointestinal tract even though clear organic changes are absent, and includes any functional decrease in the gastrointestinal tract (pharynx, esophagus, stomach, small intestine (duodenum, jejunum, ileum), large intestine). In Rome III, the definition is divided into A to H, which are functional esophagus disorder, functional gastroduodenal disorder, functional bowel disorder, functional abdominal pain syndrome, functional gallbladder and sphincter of Oddi disorder, functional anorectal disorder, infant and toddler functional disorder, and child and adolescent functional disorder.

The "gastrointestinal tract" in the present invention refers to a series of luminal organs involved in digestion from mouth cavity to anus and, for example, pharynx, esophagus, stomach, small intestine (duodenum, jejunum, ileum) and large intestine can be mentioned. The "upper gastrointestinal tract" refers to pharynx, esophagus, stomach and duodenum.

The "functional dyspepsia" in the present invention refers to diseases heretofore diagnosed as chronic gastritis or gastritis accompanied by observed delay in gastric emptying, and characteristically shows symptoms of abdominal pain, heavy stomach, heartburn and the like. According to Rome III, when one or more symptoms of bothersome postprandial fullness, early satiation, epigastric pain and epigastric burning are chronically (strictly 3 months or more) observed and structural diseases are excluded, functional dyspepsia is diagnosed.

The "gastroesophageal reflux disease" in the present invention includes erosive esophagitis and is developed by reflux of gastric acid and shows specific symptoms of heartburn, flow up of gastric acid to the mouth and the like.

Specific symptoms (indefinite complaint) of functional gastrointestinal disorders that can be prevented or improved by the prophylactic or improvement agent of the present invention include representative upper gastrointestinal indefinite complaint such as nausea, vomiting, sick feeling, heartburn, feeling of fullness in the abdomen, heavy stomach, belching, chest writhing, chest pain, gastric discomfort, anorexia and the like, lower gastrointestinal indefinite complaint such as abdominal pain, constipation, diarrhea and the like, and related complaint such as breathlessness, feeling of smothering, low incentive, pharyngeal obstruction feeling of foreign substance ("*baikakuki*" in Chinese medicine), easy fatigability, stiff neck, myotonia, mouth dryness (dry mouth thirst), tachypnea, burning sensation · cold sensation of extremities, difficulty in concentration, impatience, sleep disorder, headache, general malaise, palpitation, night sweat, anxiety, dizziness, vertigo, burning sensation, hot flash, sweating, abdominal pain, constipation, depression and the like.

The prophylactic or improvement agent of the present invention is used for the prophylaxis or improvement of a functional gastrointestinal disorder. The improvement here is a concept also including the improvement of symptoms, and prevention of the progress (aggravation) of the disease state or symptoms. In addition, prophylaxis is a concept also including prevention (prophylaxis) of the development of symptoms, and reduction of the risk of functional gastrointestinal disorders.

Glutamic acid, arginine and salts thereof to be used may be natural ones derived from animal or plant, or those obtained by a chemical synthesis method, a fermentation method or gene recombination. As the glutamic acid, an L form, a D form or a mixture thereof (e.g., racemate) can be mentioned, with preference given to an L form. Thus, aspartic acid, tricolominic acid, ibotenic acid and a salt thereof, which are amino acids similar to glutamic acid, are assumed to have an action to improve functional gastrointestinal disorders.

Arginine may be any of an L form, D form, a mixture thereof (e.g., racemate), with preference given to an L form.

As a glutamic acid salt and an arginine salt in the present invention, pharmacologically acceptable salts can be mentioned. As such salt, salts with inorganic base, salts with inorganic acid, salts with organic acid and the like can be mentioned. However, arginine glutamate is excluded from the above-mentioned salts.

As the salts with inorganic base, salts with alkali metal such as sodium, potassium, lithium and the like, salts with alkaline earth metal such as calcium, magnesium and the like, salts with ammonium and the like can be mentioned.

As the salts with inorganic acid, salts with hydrohalic acid (hydrochloric acid, hydrobromic acid, hydroiodic acid etc.), sulfuric acid, nitric acid, phosphoric acid and the like can be mentioned.

As the salts with organic acid, salts with formic acid, acetic acid, propionic acid, oxalic acid, succinic acid, maleic acid, fumaric acid, citric acid, glutamic acid, aspartic acid, hist.idine and the like can be mentioned.

Among these, a salt with an alkali metal such as sodium salt and the like are preferable, and a salt with an organic acid such as histidine salt and the like are also useful.

As glutamic acid or a salt thereof as a preferable active ingredient, glutamic acid, sodium L-glutamate, sodium D-glutamate and the like can be mentioned. Among these, glutamic acid, L-sodium glutamate and the like are preferable. As arginine or a salt thereof as a preferable active ingredient, arginine, arginine hydrochloride and the like can be mentioned.

As the active ingredient, a mixture of two or more kinds thereof can be used.

In the prophylactic or improvement agent of the present invention, the molar ratio of glutamic acid or a salt thereof and arginine or a salt thereof is generally glutamic acid:arginine = 100:1 - 1:100 (1:1 is preferably excluded). Particularly, 30:1 - 1:30 is preferable, and 10:1 - 1:10 is more preferable (1:1 is preferably excluded). Especially, 10:1 - 2:1 and 1:2 - 1:10 are preferable, and 10:1 - 2:1 is particularly preferable.

As used herein, the subject of administration includes individuals affected with functional gastrointestinal disorders (e.g., human, domestic animals and poultry such as bovine, horse, swine, sheep, dog, bird and the like, and experimental animals such as mouse, rat and the like, hereinafter the same), individuals having a risk of being affected with a functional gastrointestinal disorder, and the like.

In the present invention, the "active ingredient" refers to an ingredient capable of affording a desired prophylactic or improvement effect. While the dose of the active ingredient varies depending on the sex, age and body weight of administration subject, diet, form of administration, condition of FD, the level of risk inducing FD, condition of organic disease of the gastrointestinal tract and the like, for example, the daily dose of the active ingredient to an adult (body weight 60 kg) is generally 0.01 - 10 g, preferably 0.1 - 3 g, for glutamic acid. For arginine, it is generally 0.01 - 10 g, preferably 0.1 - 3 g.

The daily dose of the active ingredients of glutamic acid and arginine in combination is preferably 0.01 - 20 g, more preferably 0.01 - 10 g, and further preferably 0.1 - 6 g, as glutamic acid and arginine.

Such dose can be administered in one to several portions.

In the present invention, as mentioned above, functional gastrointestinal disorders can be improved by administering an effective amount of the above-mentioned active ingredient to an administration subject. In this case, the active ingredient can be administered orally, enterally or parenterally as it is or after mixing with a pharmaceutical carrier and in the form of a pharmaceutical preparation such as tablet (including sugar-coated tablet, film-coated tablet), pill, capsule, ampoule, divided powder, elixir, suspension, syrup, gum preparation, drop preparation, powder, granule, injection, suppository, sustained-release preparation and the like, in consideration of the amount of an active ingredient to be administered, condition of administration subject (e.g., patient) and the like. As the administration method, oral administration is preferable, and a sustained-release drug is more preferable. As the sustained-release form, conventional sustained-release preparations such as gel-coated preparation, multi-coated preparation and the like, gum preparation, drop preparation, localized release agent (pyloric part rupture preparation) and the like can be mentioned.

The prophylactic or improvement agent of the present invention desirably in the form of a solid preparation such as powder, granule, fine granule, tablet, capsule and the like.

In addition, an embodiment of preparation when in use wherein glutamic acid or a salt thereof and arginine or a salt thereof are individually formulated in the aforementioned molar ratio and mixed on administration, and an embodiment of simultaneous ingestion thereof on administration are also encompassed in the present invention.

The aforementioned "pharmaceutical carrier" means one which is pharmaceutically acceptable and least causes pharmacological action in the body. As a pharmaceutical carrier for oral administration, binders such as hydroxypropylcellulose, gum tragacanth, gum arabic, cornstarch, gelatin and the like; excipients such as dicalcium phosphate and the like; disintegrants such as partially pregelatinized starch, cornstarch, potato starch, alginic acid and the like; lubricants such as magnesium stearate and the like; sweetening agents such as sucrose and the like; dyes; flavorings such as orange flavor and the like; solvents such as water, ethanol, glycerol and the like; nutrients such as protein, amino acid, vitamin, lipid, glucose and the like; and the like can be used as appropriate.

Furthermore, as a pharmaceutical carrier, pharmaceutically acceptable antioxidants such as cysteine, glutathione, ascorbic acid, sodium metasulfite, sodium bisulfite and the like, and acid neutralizers such as calcium carbonate, hydroxide aluminum gel, aluminum silicate and the like can be used. One or more kinds of these can be used.

The aforementioned dosage forms of the medicament (pharmaceutical preparations) and pharmaceutical carriers are well known to those of ordinary skill in the art and, for example, the dosage forms and pharmaceutical carriers described in Reimington's Pharmaceutical Science, ed. 16 (1980) and Mack Publishing Company can be used.

The content (total amount) of glutamic acid and arginine which are active ingredients in the above-mentioned medicament (pharmaceutical preparation) is generally 0.01 - 100 wt%, preferably 0.1 - 100 wt%, more preferably 1 - 100 wt%.

The prophylactic or improvement agent of the present invention may be used in combination with other medicaments, and as such medicaments, for example, acid secretion inhibitors such as H2 receptor antagonist, proton pump inhibitor and the like, motility function improvers such as 5-HT receptor agonist, D2 antagonist and the like, antacid agents such as muscarine receptor antagonist, anti-gastrin drug, anticholinergic drug and the like, mucous membrane protectors such as teprenone, plaunotol, ornoprostil, enprostil, misoprostol, rebamipide, sucralfate, polaprezinc, azulene, egualen sodium, glutamine, aldioxa, gefarnate, ecabet sodium and the like, inflammatory colitis treating agents such as sulfasalazine, 5-ASA preparation, steroid, remicade and the like can be contained. One or more kinds of these can be contained. The prophylactic or improvement agent of the present invention and other medicaments may be formulated as a single preparation or individual preparations, and may be administered simultaneously or in a staggered manner.

The food in the present invention contains glutamic acid or a salt thereof, and arginine or a salt thereof as active ingredients (excluding arginine glutamate), and ingested for a particular object of improvement of a functional gastrointestinal disorder, or prophylaxis or improvement of functional dyspepsia or esophageal reflux. In addition, the food of the present invention may be prepared into a common food including what is called a health food. In addition, the food of the present invention may be prepared into a food with health claims, Food for specified health uses, Food with nutrient function claims, and further, a dietary supplement as defined by the food with health claims system of the Ministry of Health, Labour and Welfare. In this case, two or more kinds of glutamic acid and arginine, or salts thereof are used in a mixture. In addition, an embodiment of preparation when in use wherein glutamic acid or a salt thereof and arginine or a salt thereof are individually formulated in the aforementioned molar ratio and mixed on ingestion, and an embodiment of simultaneous ingestion thereof on administration are also encompassed in the present invention.

As the food of the present invention, the aforementioned compound may be taken as it is. For easy intake, however, general food materials, seasonings, flavoring agents and the like may be added to the above-mentioned compound and the mixture is processed into a drink, gum, powder, tablet, granule, jelly and the like before intake. In this case, for example, a tablet made of the above-mentioned compound and a disintegrant, a mixture of the above-mentioned compound and a weighting agent (protein hydrolysate, starch, casein, glucose etc.), a mixture of the above-mentioned compound and an adhesive (gum, sublingual tablet, troche) which permits intraoral sustained-release, a solution of the above-mentioned compound in a solvent capable of dissolving the compound (e.g., edible fat and oil, ethanol, water), a W/O or O/W emulsion containing the above-mentioned compound, or a mixture of the above-mentioned compound and a nutrient (e.g., protein, amino acid, vitamin, lipid, glucose etc.) can be afforded. Moreover, glutamic acid and arginine for the prophylaxis or improvement of a functional gastrointestinal disorder and prophylaxis or improvement of functional dyspepsia or esophageal reflux in the present invention can also be taken with a meal by addition thereof during the meal. For example, they can be taken by addition to an existing food such as drink, soft drink, yogurt, jelly, milk drink and the like.

When the food of the present invention is used for the aforementioned particular object, the amount of ingestion by an adult per day is generally 0.01 - 10 g, preferably 0.1 - 3 g, for glutamic acid. For arginine, it is generally 0.01 - 10 g, preferably 0.1 - 3 g.

The amount of ingestion of the active ingredients of glutamic acid and arginine in combination by an adult per day is preferably 0.01 - 20 g, more preferably 0.01 - 10 g, and further preferably 0.1 - 6 g, as glutamic acid and arginine. Such amount of ingestion can be administered in one to several portions.

The content of the above-mentioned compound in the food of the present invention is generally 0.01 - 3 wt%, preferably 0.05 - 1 wt%, more preferably 0.1 - 0.5 wt%.

In addition, the food of the present invention may be packaged in a unit serving form and the like. The unit serving form refers to a form for which the amount to be taken per serving is defined in advance, which is determined in consideration of the aforementioned ingestion amount of the active ingredients by an adult per day and the like. For example, in the case of beverages, candies, chewing gums, jellies, puddings, yogurt and the like, a given amount can be defined by packs, packages, bottles and the like; in the case of granular, powder, or slurry foods, a given amount can be defined by packages and the like, or the amount to be taken per serving may be indicated on containers and the like.

In another embodiment, a method of prophylaxis, improvement or treatment of a functional gastrointestinal disorder, comprising administration or injection of effective amounts of glutamic acid or a salt thereof, and arginine or a salt thereof (excluding arginine glutamate) by a subject affected with a functional gastrointestinal disorder is also encompassed in the present invention. The effective amount etc. are as described above.

In another embodiment, use of glutamic acid or a salt thereof, and arginine or a salt thereof (excluding arginine glutamate) for the production of an agent for the prophylaxis or improvement of functional gastrointestinal disorders is also encompassed in the present invention. The agent for the prophylaxis or improvement of functional gastrointestinal disorders is as described above.

Examples

The present invention is explained in more detail in the following by referring to Examples, which are not to be construed as limitative.

<Example 1>

To study acceleration of stomach emptying when the mixing ratio of glutamic acid and arginine is changed, the following experiment was performed.

Male SD (IGS) rats were used. A 10% casein fluid diet (1.5 mL) containing 0.05% phenol red and a test drug was orally administered, the chest was opened, and 60 minutes later, the stomach was isolated. The isolated stomach was placed in 0.1N sodium hydroxide (30 mL), homogenized and left standing for 1 hr at room temperature. Acetonitrile (1 mL) was added to 0.5 mL of the supernatant and the mixture was centrifuged (3000 rpm, 20 min). The absorbance of the supernatant was measured with an absorption spectrometer (560 nm). The gastric emptying rate was determined by the following calculation formula.

$$\text{Gastric emptying rate (\%)} = (1- \text{absorbance of test sample/absorbance of standard sample}) \times 100$$

For absorbance of a standard sample, the stomach isolated immediately after administration of 0.05% phenol red solution (1.5 mL) was used. The test drug was mixed with a phenol red solution. The glutamic acid used was sodium glutamate, and arginine used was arginine hydrochloride. When the mixing ratio of glutamic acid and arginine was 1:3, each was prepared and administered at 3 mM:9 mM (Arg3Glu9), and when the ratio was 3:1, each was prepared and administered at 9 mM:3 mM (Arg9Glu3). As a control, 10% casein liquid diet containing 0.05% phenol red was used (vehicle).

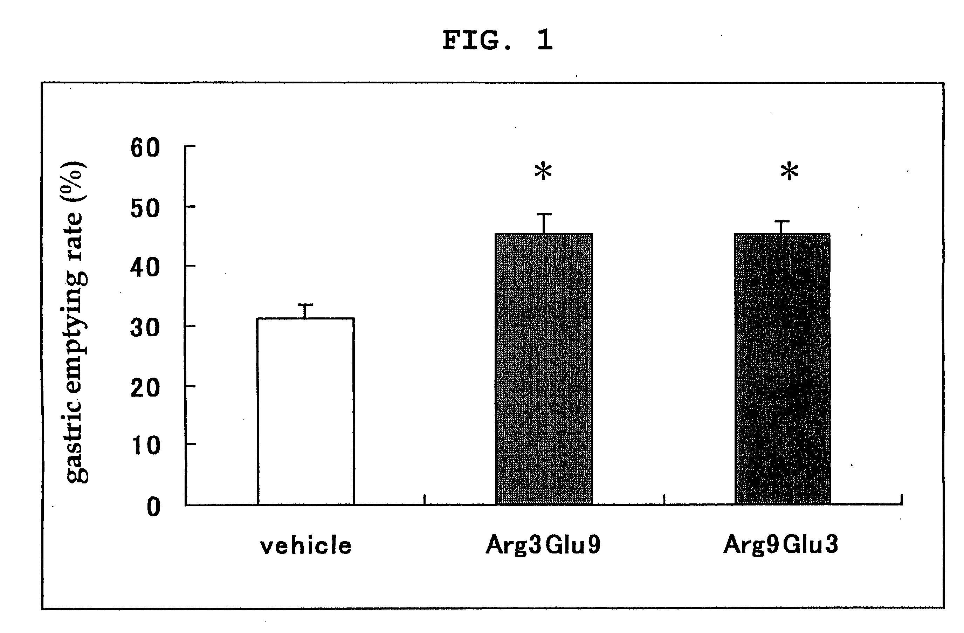

The test was performed using, after one-way analysis of variance, Dunnett's multiple comparison. *$P<0.05$. The results are shown in the Fig. 1. The number of cases in each group was 16 (15 for vehicle group alone). The vertical axis shows gastric emptying rate. It was shown that gastric emptying was accelerated at any mixing ratio of glutamic acid and arginine.

<Example 2>

To study acceleration of stomach emptying when the mixing ratio of glutamic acid and arginine is changed, the following experiment was performed.

Male SD (IGS) rat was used. 1.5 mL of 10% casein liquid diet containing 0.05% phenol red and a test drug was orally administered, the chest was opened 60 minutes later, and the stomach was isolated. The isolated stomach was placed in 0.1N sodium hydroxide (30 mL), homogenized, and left standing at room temperature for 1 hr. Acetonitrile (1 mL) was added to the supernatant (0.5 mL), and the mixture was centrifuged (3000 rpm, 20 min). The absorbance of the supernatant was measured by an absorption spectrophotometer (560 nm). Gastric emptying rate was calculated by the following calculation formula.

$$\text{Gastric emptying rate (\%)} = (1- \text{absorbance of test sample/absorbance of standard sample}) \times 100$$

For absorbance of a standard sample, the stomach isolated immediately after administration of 0.05% phenol red solution (1.5 mL) was used. The test drug was mixed with a phenol red solution. The glutamic acid used was sodium glutamate, and arginine used was arginine hydrochloride. The concentration of glutamic acid was set to 9 mM, and the concentration of arginine was changed. As a control, 10% casein liquid diet containing 0.05% phenol red was used. The tests were performed by adding arginine at 0.09, 0.03 and 0.3 mM, and 0.9, 3, 30, 90, 300 and 900 mM, in 2 days, and a vehicle group was formed for each test (vehicle and vehicle 2).

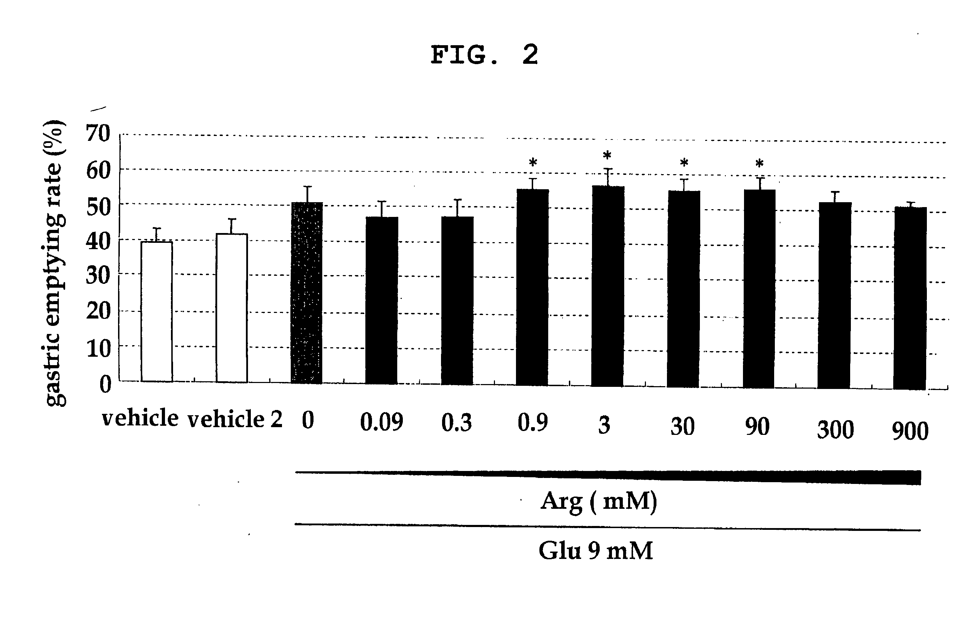

Detection was performed by Dunnett's multiple comparison after one-way analysis of variance. The group added with 0.09 mM - 0.3 mM arginine was compared with vehicle, and the group added with 0.9 mM - 900 mM arginine was compared with vehicle 2. *$P<0.05$. The results are shown in Fig. 2. The number of cases of each group was 8. The vertical axis shows gastric emptying rate (gastric emptying ability). Gastric emptying was significantly accelerated at an arginine addition concentration of 0.9 mM - 90 mM.

Formulation Example 1 (granule)

For one ingestion dose, respective components were pulverized in a grinding machine and mixed at a ratio of L-sodium glutamate (0.56 g), L-arginine hydrochloride (0.21 g) and partially gelatinized starch (1.0 g). Ethanol was added, and the mixture was kneaded in a kneading machine and granulated in an extrusion granulator to give granules.

Formulation Example 2 (granule)

For one ingestion,dose, respective components were pulverized in a grinding machine and mixed at a ratio of L-sodium glutamate (0.19 g), L-arginine hydrochloride (0.63 g) and partially gelatinized starch (1.0 g). Ethanol was added, and the mixture was kneaded in a kneading machine and granulated in an extrusion granulator to give granules.

Formulation Example 3 (tablet)

L-sodium glutamate (0.56 g), L-arginine hydrochloride (0.21 g) and hydroxypropylcellulose (0.2 g) were added per tablet and granulated. After drying, the granules were sieved, mixed and pressed to give tablets.

Formulation Example 4 (tablet)

L-sodium glutamate (0.19 g), L-arginine hydrochloride (0.63 g) and hydroxypropylcellulose (0.2 g) were added per tablet and granulated. After drying, the granules were sieved, mixed and pressed to give tablets.

While some of the embodiments of the present invention have been described in detail in the above, it will, however, be evident for those of ordinary skill in the art that various modifications and changes may be made to the particular embodiments shown without substantially departing from the teaching and advantages of the present invention. Accordingly, such modifications and changes are encompassed in the spirit and scope of the present invention as set forth in the appended claims.

This application is based on application No. 2008-061769 filed in Japan, the contents of which are incorporated hereinto by reference.

**Claims**

1.  An agent for the prophylaxis or improvement of a functional gastrointestinal disorder, comprising glutamic acid or a salt thereof, and arginine or a salt thereof, excluding arginine glutamate, as active ingredients.

2.  The agent according to claim 1, wherein the functional gastrointestinal disorder is an upper gastrointestinal dysfunction.

3.  The agent according to claim 2, wherein the upper gastrointestinal dysfunction is a functional dyspepsia or gastroesophageal reflux disease.

4.  The agent according to any one of claims 1 to 3, wherein a molar ratio of the glutamic acid or a salt thereof and arginine or a salt thereof is 30:1 - 1:30.

5.  The agent according to claim 4, wherein the molar ratio is 10:1 - 1:10.

6.  The agent according to claim 4, wherein the molar ratio is 10:1 - 2:1 or 1:2 - 1:10.

7.  The agent according to claim 4, wherein the molar ratio is 10:1 - 2:1.

8.  A medicament comprising the agent according to any one of claims 1 to 7.

9.  A food comprising the agent according to any one of claims 1 to 7.

10. The food according to claim 9, which is a food with health claims, food for specified health uses, food with nutrient function claims or a dietary supplement.

11. A method of preventing or improving a functional gastrointestinal disorder, comprising administering an effective amounts of glutamic acid or a salt thereof, and arginine or a salt thereof, excluding arginine glutamate, to a subject affected with a functional gastrointestinal disorder.

12. A method of treating a functional gastrointestinal disorder, comprising administering effective amounts of glutamic acid or a salt thereof, and arginine or a salt thereof, excluding arginine glutamate, to a subject affected with a functional gastrointestinal disorder.

13. Use of glutamic acid or a salt thereof, and arginine or a salt thereof, excluding arginine glutamate, for producing the agents according to claims 1 to 7.

# FIG. 1

FIG. 2

EP 2 263 666 A1

## INTERNATIONAL SEARCH REPORT

International application No.

PCT/JP2009/054697

A. CLASSIFICATION OF SUBJECT MATTER
*A61K31/198*(2006.01)i, *A23L1/305*(2006.01)i, *A61P1/04*(2006.01)i, *A61P1/06* (2006.01)i

According to International Patent Classification (IPC) or to both national classification and IPC

B. FIELDS SEARCHED

Minimum documentation searched (classification system followed by classification symbols)
A61K31/198, A23L1/305, A61P1/04, A61P1/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | JP 2003-530411 A (SCIETE DES PRODUITS NESTLE S.A.), 14 October, 2003 (14.10.03), Particularly, Claims; Par. Nos. [0003], [0049], [0051] to [0053] & WO 2001/78532 A1 & EP 1251844 A & US 2005/80137 A1 | 1-10,13 |
| X | WO 2006/30980 A1 (Ajinomoto Co., Inc.), 23 March, 2006 (23.03.06), Particularly, Claims; examples & EP 1806134 A1 & US 2007/0218112 A1 & JP 2008-115185 A | 1-10,13 |

| ☒ Further documents are listed in the continuation of Box C. | ☐ See patent family annex. |
|---|---|

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May, 2009 (25.05.09) | 02 June, 2009 (02.06.09) |

| Name and mailing address of the ISA/ Japanese Patent Office | Authorized officer |
|---|---|
| Facsimile No. | Telephone No. |

Form PCT/ISA/210 (second sheet) (April 2007)

**INTERNATIONAL SEARCH REPORT**

| | International application No. |
|---|---|
| | PCT/JP2009/054697 |

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2006/001492 A1  (Ajinomoto Co., Inc.),<br>05 January, 2006 (05.01.06),<br>Particularly, Claims; examples<br>& US 2007/0218109 A1    & EP 1767201 A1<br>& WO 2006/001492 A1 | 1-10,13 |
| Y | JP 2004-521898 A  (Kamm, Michael Albert),<br>22 July, 2004 (22.07.04),<br>Particularly, Claims<br>& US 2004/0063684 A1    & EP 1357905 A<br>& WO 2002/062324 A2    & CA 2437380 A | 1-10,13 |
| Y | Masahiro OCHI et al., "Kinosei Shokaki Shikkan<br>no Kiso to Rinsho NO Gosei Sogaizai ni yoru I<br>Haishutsu Chien Model to FD Shorei ni Taisuru<br>Kakushu Yakuzai Koka", Gastroenterology, 2006,<br>Vol.43, No.6, pages 503 to 507 | 1-10,13 |
| Y | Toshitaka Kido et al., Effects of _Rikkunshi-to_,<br>a Traditional Japanese Medicine, on the Delay<br>of Gastric Emptying Induced by $N^G$-Nitro-L-<br>arginie, Journal of Pharmacological Sciences,<br>2005, Vol.98, No.2, p.161-167 | 1-10,13 |
| X | JP 2000-93123 A  (Q.P. Corp.),<br>04 April, 2000 (04.04.00),<br>Particularly, Claims; examples<br>(Family: none) | 9,10 |
| X | JP 4-112825 A  (The Institute of Physical and<br>Chemical Research, Nippon Steel Corp.),<br>14 April, 1992 (14.04.92),<br>Particularly, Claims<br>(Family: none) | 9,10 |
| X | JP 2000-63284 A  (Terumo Corp., Morinaga Milk<br>Industry Co., Ltd.),<br>29 February, 2000 (29.02.00),<br>Particularly, Claims; examples<br>(Family: none) | 9,10 |
| X | JP 58-88323 A  (The Food Science Institute<br>Foundation),<br>26 May, 1983 (26.05.83),<br>Particularly, Claims; examples<br>(Family: none) | 9,10 |

Form PCT/ISA/210 (continuation of second sheet) (April 2007)

<div style="text-align: center;">

**INTERNATIONAL SEARCH REPORT**

</div>

| International application No. |
|---|
| PCT/JP2009/054697 |

| Box No. II      Observations where certain claims were found unsearchable (Continuation of item 2 of first sheet) |
|---|

This international search report has not been established in respect of certain claims under Article 17(2)(a) for the following reasons:

1. ☒ Claims Nos.: 11, 12
    because they relate to subject matter not required to be searched by this Authority, namely:

    Claims 11 and 12 pertain to method for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

| Box No. III      Observations where unity of invention is lacking (Continuation of item 3 of first sheet) |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2006030980 A1

• JP 2008061769 A

**Non-patent literature cited in the description**

• Reimington's Pharmaceutical Science. Mack Publishing Company, 1980, vol. 16